# EUROPEAN PATENT SPECIFICATION

(11) **EP 2 033 529 B1**
(45) Date of publication and mention of the grant of the patent: **11.12.2019**
(21) Application number: 08167964.9
(22) Date of filing: 25.10.2004
(51) Int. Cl.: A61K 35/745, A61K 31/733, A61K 31/702, C12Q 1/689, A23J 1/20, A61K 31/7016, A61K 31/715, A23L 33/00, A23L 33/135, A23L 33/19, A23L 33/21

(54) **Synbiotic composition for infants**
Synbiotische Zusammensetzung für Kleinkinder
Composition symbiotique pour nourrissons

(30) Priority: 24.10.2003 EP 03078374
(43) Date of publication of application: 11.03.2009
(62) Divisional of application: 04793673.7
(73) Proprietor: N.V. Nutricia, 2712 HM Zoetermeer (NL)
(72) Inventor: Speelmans, Gelske, 6708 LW Wageningen (NL); Knol, Jan, 6708 SM Wageningen (NL); Haarman, Monique, 6706 KS Wageningen (NL); Garssen, Johan, 3434 SG Nieuwegein (NL)
(74) Representative: Nederlandsch Octrooibureau

(56) References cited:
- EP-A- 0 856 259
- EP-A- 0 904 784
- EP-A- 1 175 905
- EP-A1- 1 010 753
- EP-A1- 1 062 873
- WO-A-03/013559
- DE-A- 10 008 279
- DE-A- 10 206 995
- DE-A- 19 836 339
- US-A1- 2002 015 990
- US-A1- 2003 031 659
- PATENT ABSTRACTS OF JAPAN vol. 1999, no. 2, 26 February 1999 (1999-02-26) & JP 10, 309178, A, (WAKAMOTO PHARMACEUT CO LTD), 24 November 1998 (1998-11-24)
- FAMULARO G ET AL: "TRADITIONAL AND HIGH POTENCY PROBIOTIC PREPARATIONS FOR ORAL BACTERIOTHERAPY", BIODRUGS, AUCKLAND, NZ, vol. 12, no. 6, 1999, pages 455-470, XP009001219, ISSN: 1173-8804
- MORO G ET AL: "Dosage-Related Bifidogenic Effects of Galacto- and Fructooligosaccharides in Formula-Fed Term Infants", JOURNAL OF PEDIATRIC GASTROENTEROLOGY AND NUTRITION, RAVEN PRESS, NEW YORK, NY, US, vol. 34, no. 3, March 2002 (2002-03), pages 291-295, XP008029577, ISSN: 0277-2116
- MORISHITA Y ET AL: "Galactooligosaccharide in combination with Bifidobacterium and Bacteroides affects the population of Clostridium perfringens in the intestine of gnotobiotic mice.", NUTRITION RESEARCH, vol. 22, no. 11, November 2002 (2002-11), pages 1333-1341, XP002295913, ISSN: 0271-5317
- ISOLAURI E ET AL: "PROBIOTICS IN THE MANAGEMENT OF ATOPIC ECZEMA", CLINICAL AND EXPERIMENTAL ALLERGY, BLACKWELL SCIENTIFIC PUBLICATIONS, LONDON, GB, vol. 30, November 2000 (2000-11), pages 1604-1610, XP001064231, ISSN: 0954-7894
- ISOLAURI E ET AL: "PROBIOTICS: EFFECTS ON IMMUNITY", AMERICAN JOURNAL OF CLINICAL NUTRITION, BETHESDA,MD, US, vol. 73, no. SUPPL, February 2001 (2001-02), pages 444S-450S, XP000984966, ISSN: 0002-9165
- KANAMORI YUTAKA ET AL: "Combination therapy with Bifidobacterium breve, Lactobacillus casei, and galactooligosaccharides dramatically improved the intestinal function in a girl with short bowel syndrome: A novel synbiotics therapy for intestinal failure", DIGESTIVE DISEASES AND SCIENCES, vol. 46, no. 9, September 2001 (2001-09), pages 2010-2016, XP009036227, ISSN: 0163-2116
- LAMOUREUX L ET AL: "PRODUCTION OF OLIGOSACCHARIDES IN YOGHURT CONTAINING BIFIDOBACTERIA AND YOGURT CULTURES", JOURNAL OF DAIRY SCIENCE, AMERICAN DAIRY SCIENCE ASSOCIATION. CHAMPAIGN, ILLINOIS, US, vol. 85, no. 5, May 2002 (2002-05), pages 1058-1069, XP001124200, ISSN: 0022-0302
- YASUI H ET AL: "Immunomodulatory function of lactic acid bacteria", ANTONIE VAN LEEUWENHOEK, KLUWER ACADEMIC PUBLISHERS, DO, vol. 76, 1 January 1999 (1999-01-01), pages 383-389, XP002538609, ISSN: 1572-9699, DOI: 10.1023/A:1002041616085
- LEON PROSKY ET AL: "Determination of insoluble, soluble, and total dietary fiber in foods and food products: interlaboratory study", JOURNAL / ASSOCIATION OF OFFICIAL ANALYTICAL CHEMISTS,, vol. 71, 1 September 1988 (1988-09-01), pages 1017-1023, XP009195037,

## Description

### TECHNICAL FIELD OF THE INVENTION

The present invention relates to preparations comprising a probiotic and a prebiotic for infants, in particular for non-breast-fed infants.

### BACKGROUND OF THE INVENTION

Infants are devoid of intestinal flora at birth. As a result of contact with the mother during birth and subsequent breast feeding, the intestinal flora rapidly develops and increases. During the development, the intestinal flora is still immature and its equilibrium is fragile and quickly prone to changes and thus to the occurrence of diseases and affections in the presence of pathogens. Breast-fed infants are known to be less afflicted by infections or diseases than non-breast-fed infants. Hence, breast-fed babies have less gastro-intestinal infections in terms of both incidence and duration, less atopic diseases such as allergy, eczema, allergy induced asthma, and less constipation than non-breast-fed infants.

Generally, the intestinal flora of breast-fed infants is primarily composed of bifidobacteria and lactic acid bacteria. Breast milk contains human milk oligosaccharides (HMO), which are a growth factor for bifidobacteria in the intestine of infants. The flora of formula-fed infants is more diverse and contains in general more *Bacteroides*, *Clostridium* and *Enterobacteriaceae* species. Formula-fed infants have about one-tenth to roughly two-third the number of bifidobacteria of breast-fed infants. Bifidobacteria are considered to be important in maintaining a well-balanced intestinal microbiota and it has been postulated that bifidobacteria have several health-promoting effects, including the prevention and/or treatment of diarrhea and intestinal infections. Furthermore, bifidobacteria have been shown to play a role in the immune system of the host.

The intestinal flora of infants may be modified by nutritional changes in the diet, like consumption of probiotics or prebiotics. As an example of the probiotics approach, EP-A-0,904,784 describes the administration of a mixture of micro-organism strains, including *Bifidobacterium* strains. However, a problem associated therewith is that the mixture of microbes, while providing some health benefit, may also have a deleterious effect on the still immature intestinal flora of non-breast-fed infants due to its broad spectrum of action. Further, many probiotic supplements have a short shelf-life and contain too low a number of living microorganisms, thereby failing to provide the expected probiotic effects.

Prebiotics are defined as non-digestible food ingredients that selectively stimulate the growth and/or activity of one or more bacteria in the colon and thereby beneficially affect the host (Gibson and Roberfroid, J. Nutr. 125:1401-14121995). A preferable way to improve the intestinal flora of bottle-fed babies is to selectively stimulate the bifidobacteria already present in the bottle-fed infant's intestine by specific non-digestible oligosaccharides, i.e. prebiotics. Also, mixtures of oligosaccharides and polysaccharides have been proposed as prebiotics, e.g. in WO 00/08948. One example is the combination of galacto-oligosaccharide with fructopolysaccharides. The bifidobacteria level in infants receiving a formula containing these prebiotics has been shown to be elevated in comparison with a standard formula (see e.g. Moro et. al. J. Pediatr. Gastroenterol. Nutr. 34:291-295, 2002).

The approach up to now was to promote bifidobacteria in general, i.e. on the genus level. The genus *Bifidobacterium* consists of many different species, which differ in metabolism, enzyme activity, oligo- and polysaccharide utilisation, cell wall composition, and interaction with the host's immune system. It therefore can be expected that not every species of *Bifidobacterium* has the same functional effect on the infant. Examples of different *Bifidobacterium* species are *B. longum, B. breve, B. infantis, B. adolescentis, B. bifidum, B. animalis,* and *B. dentium. B. adolescentis* is more prevalent in the flora of adults, and is less common in faeces of healthy infants and babies. *B. animalis* /*B. lactis* is not naturally occurring in humans, and *B. dentium* is a pathogenic bacterium. In healthy infants the bifidobacterial flora is mainly composed of *Bifidobacterium infantis*, *B. breve* and *B. longum.* Kalliomaki et. al. (Curr Opin Allergy Clin Immunol. 2003 Feb;3(1):15-20, and references cited therein), reported that allergic infants harbour an adult-like *Bifidobacterium* flora whereas a typical infant *Bifidobacterium* flora was shown in healthy infants, indicating a correlation between the occurrence of certain *Bifidobacterium* species and the chance of developing allergy. These results indicate that the stimulation of the genus *Bifidobacterium* in the baby's colon may not be sufficient. It is the aim to achieve a flora in bottle-fed infants that is reminiscent to the flora of breast fed babies on a species level.

For the purpose of the present invention, "breast-fed infants" refers to infants which are exclusively fed with human breast milk. "Non- or partially breast-fed infants" means infants which are not or not exclusively receiving human breast milk. This definition includes those infants which are receiving at least the content of a bottle per day, i.e. at least 80 ml of formula milk per day, the rest, if any, of the nutrition being provided from solid nutrition or liquid nutrition such as breast milk, i.e. partly-breast-fed infants.

EP 1 062 873 A1 describes the use of a combination of two different carbohydrate components and explains a benefit thereof in case of diaper rash. EP 904 784 A1, Isolauri et al. Am J Clin Nutr 2001, 73:444S-450S and Yasei et. al. Antonie van Leeuwenhoek, 1999, 76:383-389 describe immunomodulation by probiotics. Moro et al, 2002, J Pediatr Gastroenterol Nutr 43:291-295 discloses dosage-related bifidogenic effects of galacto- and fructooligosaccharides in formula fed term infants.

### SUMMARY OF THE INVENTION

It was been found that the increase in the level of *Bifidobacterium* using mixtures of non-digestible carbohydrates also regulates the *Bifidobacterium* population to a more infant-like population, i.e. low in *B. catenulatum, B. pseudocatenulatum* and *B. adolescentis*, whereas infants fed with a standard formula exhibit a more adult-like flora, that is more predominant in *B. catenulatum, B. pseudocatenulatum* and *B. adolescentis.* It was also found that the *Bifidobacterium* population in such prebiotic-fed infants was still deficient in one particular microorganism, namely *Bifidobacterium breve.*

Accordingly, a preparation comprising *Bifidobacterium breve* and a mixture of non-digestible carbohydrate prebiotics was found beneficial and very suitable for regulating the *Bifidibacterium* population on a species level in the gastro-intestinal tract of infants. Furthermore, it was surprisingly found that addition of other *Bifidobacterium* species than *B. breve* species is not necessary, as they are sufficiently regulated by the preparation as such.

### DETAILED DESCRIPTION OF THE INVENTION

### Preparation

### 1) Bifidobacterium breve

*Bifidobacterium breve* is an essential ingredient of the present invention. This bacterium has been found by the Applicant's method of detection as being present in limited quantities in non-breast-fed infants. Accordingly, the administration of this bacterium with the carbohydrate mixture enables the normalisation of the *Bifidobacterium* species population to a level equivalent to that present in the gastrointestinal tract of breast-fed infants.

Preferred *Bifidobacterium breve* strains are those selected from isolates from the faeces of healthy breast-fed infants. Typically, these are commercially available from producers of lactic acid bacteria, but they can also be directly isolated from faeces, identified, characterised and produced. Examples of commercially available *B. breve* are *B. breve* Bb-03 from Rhodia, *B. breve* MV-16 from Morinaga, and *B. breve* from Institut Rosell, Lallemand, but *B. breve* can also be obtained from culture collections such as DSM 20091, and LMG 11613.

The amount of *B. breve* in the preparation of the invention can be based on the total amount of soluble non-digestible carbohydrates, and is preferably from 10⁷ to 10¹¹, more preferably from 10⁸ to 10¹⁰ cfu of the bacteria per g of the total of these carbohydrates. When the preparation is used as a supplement, the *Bifidobacterium breve* is most preferably present in the supplement in an amount of from 1x10⁶ to 1.5x10¹¹ cfu/g, preferably from 3x10⁷ to 5x10¹⁰ cfu/g, more preferably from 5x10⁸ to 1x10¹⁰ cfu/g. When the preparation is used as a (complete) infant nutrition, the *B. breve* is most preferably present in the nutrition in an amount of from 1x10⁴ to 1x10¹⁰ cfu/g, preferably from 5x10⁶ to 3x10⁹ cfu/g, more preferably from 1x10⁷ to 5x10⁸ cfu per g of the infant nutrition. These concentration are chosen in such a way that the daily dose is about 1x10⁶ to 1.5x10¹¹ cfu/g, preferably from 3x10⁷ to 5x10¹⁰ cfu/g, more preferably from 5x10⁸ to 1x10¹⁰ cfu/g.

### 2) Mixture of non-digestible carbohydrate prebiotics

A mixture of non-digestible carbohydrate prebiotics is also an essential element of the invention. By "non-digestible", it is meant that that the carbohydrates remain undigested in the gastrointestinal tract and reach the large intestine unresorbed.

For the purpose of the invention, the mixture of non-digestible carbohydrates contains at least two different, soluble carbohydrate components A and B, which remain undigested in the gastrointestinal tract and reach the large intestine unresorbed. The carbohydrate mixtures according to the present invention may also consist exclusively of these two carbohydrate components A and B.

In the mixture of at least two non-digestible soluble carbohydrate components A and B, at least 50%, preferably at least 75%, of the total non-digestible soluble carbohydrates of components A and B is selected from disaccharides to eicosasaccharides (polysaccharides having 20 monosaccharide units); the remainder may be non-digestible monosaccharides and non-digestible polysaccharides which are longer than 20 units. It is also preferred that more than 95%, preferably more than 98% of the total soluble non-digestible carbohydrates has a chain length of no more than 100 units. Where percentages and averages are mentioned in this description, percentages and averages by weight are meant, unless it is evident that another basis is meant or when otherwise specified.

The carbohydrates of components differ:
(i) in the (average) number of monosaccharide units of the carbohydrate, component A having an average chain length which is at least 5 monosaccharide units lower than the average chain length of component B;
(ii) in the structure of the monosaccharide units of the carbohydrate, component A being built up from different structural units from component B; where A and/or B are built up from repeating combinations of different monosaccharides units, for example in the case of galactomannans and arabinogalactans, at least 50% of the monosaccharide units of the two components should be different (in the above example either or both should have less than 50% anhydrogalactose units);

Preferably, component A is selected from indigestible monosaccharides up to hexasaccharides of the same carbohydrate structure, and component B is selected from heptasaccharides and higher polysaccharides of the same carbohydrate structure. Carbohydrate component A thereby consists of at least one non-digestible monosaccharide or at least one non-digestible oligosaccharide. With oligosaccharides it is understood those comprising 2 up to and including 6 monosaccharide units. Carbohydrate component A may also, and preferably, be formed by a mixture of two or more of the mentioned saccharides. It may therefore be comprised of any number of various monosaccharides and/or oligosaccharides of that kind, i.e. of the same structure.

According to this preferred embodiment, carbohydrate component B consists of at least one polysaccharide comprising 7 or more monosaccharide units. With polysaccharides it is understood those starting from heptasaccharide (e.g. heptasaccharide, octasaccharide, nonasaccharide, decasaccharide, etc.). There is no specific upper limit to the chain length of polysaccharides, and they may be as long as several hundreds or even thousands of monosaccharide units. However, chain lengths of more than 100 (about 16 kD), and especially those of more than 700 (about 100 kD) are less preferred according to the invention. Preferably, component B does not contain more than 5% or even not more than 2% of homologues having more than 100 monosaccharide units. Carbohydrate component B may also be comprised of only one polysaccharide of that kind or, preferably, of two or more polysaccharides of different length of that kind, i.e. of the same structure. Carbohydrate component A represents up to 95 wt% of the sum of carbohydrate component A and carbohydrate component B (A + B = 100 wt%). Carbohydrate component B represents 5 to 20 wt% of the sum of carbohydrate component A and carbohydrate component B. Component A constitutes 95 to 80 wt.% and in particular 95 to 90 wt%, and component B 5 to 20 wt.% and in particular 5 to 10 wt% of the carbohydrates present *in toto*, with A + B = 100 wt%.

As soluble carbohydrates in the sense of the present invention are understood those that are at least 50% soluble, according to a method described by L. Prosky et al, J. Assoc. Anal. Chem 71: 1017-1023, 1988. The method concerns modified AOAC methods 43.A14 - 43.A.20 wherein the concentration of buffers were modified and hydrochloric acid is substituted for phosphoric acid. More specifically, the buffers are according to AOAC 43.A19, except use 0.275N NaOH instead of 0.171N NaOH, 0.325M HCl instead of 0.205M H3P04, and 0.08M phosphate buffer instead of 0.05M phosphate buffer.

At least 80 wt% of the carbohydrates or saccharides out of the sum of carbohydrate component A and B thereby have a prebiotic effect. Preferably, at least 80 wt% of the carbohydrates belonging to carbohydrate component A, and also at least 80 wt% of those belonging to carbohydrate component B, have a prebiotic effect. In other words, preferably at least 80 wt% each of the carbohydrates or saccharides out of carbohydrate components A and B, are intended to reach the large intestine in an undigested (hence not resorbable in the small intestine) manner. In other words, these carbohydrates or saccharides of carbohydrate components A and B in the gastrointestinal tract are neither resorbed and digested in the stomach nor in the small intestine, but reach the large intestine as such.

With a prebiotically active carbohydrate according to the present invention it is understood a carbohydrate, which reaches the large intestine undigested (hence not resorbable in the small intestine), and there, it selectively encourages the growth and/or the activity of one or of a restricted number of bacterial species in the intestine, and consequently promotes health. This prebiotic effect of such carbohydrates and their specific mechanisms are described in detail in "G.F. Gibson & M.B. Roberfroid, J.Nutr. 1995; 125: 1401-1412", whereto explicit reference is made herewith, and of which the disclosure is included in the present document.

The proportion of the non-prebiotically active carbohydrates or saccharides of carbohydrate components A and B therewith amounts to a maximum of 20 wt%. These carbohydrates or saccharides refer to those which are actually soluble but can be excreted in an undigested form. These carbohydrates can exercise a physical effect in that they increase, for example, the volume of the faeces or prompt a water adsorption.

For the assessment of the proportion determining the carbohydrate components A and B in a dietary or pharmaceutical product, the following steps are carried out. In a first stage, all soluble carbohydrates are extracted from the product by means of water. Fats and proteins are removed from the extract. In a second stage, the soluble carbohydrates or the extract, respectively, are digested by means of human enzymes, e.g. human amylase, human pancreatic juice or small intestine ciliated border preparations. The yield of non-digested carbohydrates (except for the *in vivo* resorbable monosaccharides obtained in this *in vitro* experiment), constitutes the two carbohydrate components A and B. 80 % thereof are supposed to be prebiotically active.

Hence, the carbohydrate mixtures to be used in the preparation of the invention are those, wherein the carbohydrates, which are soluble and undigested in the sense described above, fulfil the herein specified criteria and constitute the carbohydrate components A and B.

By means of a selective combination of oligosaccharides and polysaccharides, and consequently the simultaneous presence of carbohydrate components A and B, the health-promoting microorganisms in the large intestine may be promoted and/or pathogenic microorganisms may be suppressed by an essentially higher efficiency than would be the case with only one of said carbohydrate components. Thus, it is possible with the administration of the carbohydrate combination, to achieve a very rapid restitution of a normal large intestinal flora, to maintain the same or to prophylactically prevent an alteration of the intestinal flora during situations of stress, and thus to influence the bacterial colonisation of the large intestine in a way, which is more efficient than the one with the previously used carbohydrates.

At least 80 wt% of carbohydrate component A as well as of carbohydrate component B consist of carbohydrates, which are bifidogenic and/or which promote lactic acid bacteria. Due to such a combination of oligosaccharides and polysaccharides having said properties, the growth of the lactic acid bacteria may surprisingly be promoted in an essentially stronger manner than would be the case with oligosaccharides or polysaccharides alone. Not only lactic acid bacteria are thereby promoted, which are naturally present in the intestine, but also the growth of those is promoted - optionally even in a selective manner - which are introduced exogenously.

Apart from this indirect action via the bacteria themselves and their metabolites such as organic acids (acetate, lactate, etc.), pH effects and stimulation of colonozytes, direct physical effects such as peristalsis, water content, quantity of faeces, mechanical action upon the intestinal *mucosa*, are likewise positively influenced.

Thus, the carbohydrate mixtures dispose not only of a nutritive effect but also of a wide spectrum of activities. In addition to the above-described biological effects, the following may also be achieved by means of the inventive mixtures: stabilisation of natural microflora, prevention of pathogenic substances/organisms such as toxins, viruses, bacteria, fungi, transformed cells and parasites from adhering, dissolution of complexes of toxins, viruses, bacteria, fungi and other pathogens having endogenous cells, as well as their elimination from the body, and an acceleration of wound healing.

Thus, the mixtures are suitable for the prophylaxis and/or the treatment of symptoms or diseases occurring in conjunction with a disturbed intestinal flora, for example, as a consequence of the association or adhesion of the mentioned substances and organisms with or on epithelia or other endogenous cells.

The chain length of the polysaccharide of component B, or the weight-average chain length in case of a mixture of polysaccharides, is at least five units longer than the chain length of the oligosaccharide of component A or the weight-average of a mixture of oligosaccharides. Preferably, the average chain length of the oligosaccharides A is between 2 and 6 units, and the average chain length of the polysaccharides B is between 7 and 30, more preferably between 8 and 20. Where both oligosaccharides and polysaccharides of the same structure are present, the carbohydrates of this structure are considered as component A when the weight-average chain length is below 6.5 and the individual members having a chain length of 7 and higher are not counted with the component A; on the other hand, they are considered as component B when the weight-average chain length is above 6.5 and then the individual members having a chain length of 6 and lower are not counted with the component B.

A preferred carbohydrate mixture is composed of galacto-oligosaccharide and inulin.

According to the invention 80 to 100 wt% of carbohydrate components A belong to the group of galacto-oligosaccharides and 80 to 100 wt% of the carbohydrate components B belong to the group of fructo-polysaccharides. For the production of the carbohydrate mixtures, carbohydrates and carbohydrate mixtures presently known and used in particular for the production of foods or food products can be used. It is also possible to use raw materials previously modified in a technical way. The preparation of the mixtures may thereby ensue by means of a simple blending of the correspondingly selected carbohydrates or oligosaccharides with polysaccharides or the carbohydrate mixtures. The initial components must thereby be so mixed with one another that the parameters are respected with the finished mixtures.

Non-digestible carbohydrates according to the present invention are typically administered at a daily dose of 0.5 to 30 g, preferably 2 to 15 g, more preferably 3 to 9 g.

One preferred mode of administration of the preparation is as a supplement. The supplement is suited for infants which are non-breast-fed or partly breast-fed, including non- or partly breast-fed prematurely born babies and non- or partly- breast-fed maturely born babies.

The preparation may also be used as an infant nutrition. In this case, the invention infant nutrition further comprises one or more ingredients selected from digestible carbohydrate, a lipid source, protein source, and mixtures thereof.

### 3) Other components

Apart from the carbohydrate components A and B, other carbohydrates may be present as well. Amongst those are 1) the digestible carbohydrates, which are digestible as described above, and 2) the insoluble carbohydrates, which are resorbable/digestible or even not resorbable/digestible. Typical insoluble non-digestible carbohydrates for use in the infant nutrition supplement are soy polysaccharides, and resistant starch, cellulose and hemicellulose; more preferably they are selected from soy polysaccharides and resistant starch.

Typical soluble and digestible carbohydrate for use in the infant nutrition supplement are selected from maltodextrins, starch, lactose, maltose, glucose, fructose, and sucrose and other mono- and disaccharides, and are more preferably selected from maltodextrin, lactose, maltose, glucose, fructose, sucrose, and mixtures thereof.

These carbohydrates enumerated *sub* 1) and 2), may be present as such in any arbitrary quantity in addition to the carbohydrate components A and B, in each case depending on the desired final product. Preferably, the insoluble carbohydrates constitute 0 to 10 wt% of the carbohydrate mixtures.

Typical ingredients for use as a lipid source for use in the infant nutrition supplement may be any lipid or fat which is suitable for use in infant formulas. Preferred lipid sources include milk fat, safflower oil, egg yolk lipid, canola oil, olive oil, coconut oil, palm oil, palm kernel oil, palm olein, soybean oil, sunflower oil, fish oil, and microbial fermentation oil containing long-chain polyunsaturated fatty acids. These oils may be in the form of high oleic form such as high oleic sunflower oil and high oleic safflower oil. The lipid source may also be in the form of fractions derived from these oils such as palm olein, medium chain triglycerides (MCT), and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

For pre-term formulas, the lipid source preferably contains medium chain triglycerides, preferably in an amount of 15% to 35% by weight of the lipid source.

The lipid source preferably has a molar ratio of n-6 to n-3 fatty acids of 5:1 to 15:1, preferably from 8:1 to 10:1.

When present, it is preferred that the lipid are present at levels of from 20% to 40% by weight of the composition or as 0.8 to 1.5 g/100 kJ in an infant formula.

The proteins that may be utilised in the nutritional products of the invention include any protein or nitrogen source suitable for human consumption. Examples of suitable protein sources for use in infant formula typically include casein, whey, condensed skim milk, non-fat milk, soy, pea, rice, corn, hydrolysed protein, free amino acids, protein sources which contain calcium in a colloidal suspension with the protein and mixtures thereof. It is preferred for use herein that the protein are in hydrolysate form, thereby reducing the risk of allergy in such infant. Commercial protein sources are readily available and known to one practicing the art.

Typically, in the milk-based infant formula hydrolysates 100 % hydrolysed whey protein from cow's milk is present. In other milk-based infant formulae the ratio of casein/whey typically is between 1.8:0.3-3-0.

When present, it is preferred that the protein source is present at a levels of from 9% to 19% by weight of the composition. When used as an infant formula, the protein source is preferably present in an amount of from 0.45 to 1.0 g /100 kJ.

A nutritionally complete formula preferably contains all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B, vitamin B2, vitamin B6, vitamin B12, vitamin E, vitamin K, vitamin C, vitamin D, folic acid, inositol, niacin, biotin, pantothenic acid, choline, calcium, phosphorous iodine, iron, magnesium, copper, zinc, manganese, chloride, potassium, sodium selenium, chromium, molybdenum, taurine, and L- carnitine. Minerals are usually added in salt form. The presence and amounts of specific minerals and other vitamins will vary depending on the intended infant population.

If necessary, the infant formula may contain emulsifiers and stabilisers such as soy lecithin, citric acid, esters of mono and di-glycerides, and the like. This is especially provided if the formula is to be provided in liquid form.

The infant formula may optionally contain other substances which may have a beneficial effect such as (non-carbohydrate) fibres, lactoferrin, immunoglobulins, nucleotides, nucleosides, and the like.

### Applications

The preparations according to the invention have been found to be particularly useful in the normalisation of the *Bifidobacterium* population according to the species distribution in breast-fed infants, considered as "standard", in the gastro-intestinal tract of infants which were non- or partly breast-fed, in particular those which are prematurely born babies, maturely born babies, as well as infants which are in the adaptation period to solid food. The preparation of the invention is also suitable for infants changing from breast to bottle feeding.

The preparations of the invention have also been found particularly useful for the prevention or treatment of an immune condition. This immune condition is believed to be the result of the difference in the composition of the *Bifidobacterium* species in the gastrointestinal tract of these non- or partly breast-fed infants when compared to that of breast-fed infants.

Typically, such immune conditions include conditions selected from allergy, atopic dermatitis, eczema, asthma, atopy, allergic rhinitis, food hypersensitivity, diapers rashes, diarrhoea, and mixtures thereof.

Accordingly, the invention provides the use of the preparation for the prevention or treatment of immune conditions mediated diapers rashes.

Advantageously, the preparation has been found beneficial for inhibiting the infiltration of eosinophils, neutrophils and mononuclear cells in allergic lesions, and/or inhibiting the Th2 type immune response and/or stimulating the Th1 mediated immune response.

### Example 1: Validation of the developed probes and primers for bifidobacteria

The bacterial strains used to validate the assays for the relative quantification of the different *Bifidobacterium* species are listed in Table 2.

**.Table 2**

| Bacterial strains and origins used for the development of the 5' nuclease assays | | | |
|---|---|---|---|
| **Strain** | **Origin^{a}** | | |
| *Bifidobacterium* strains | | | |
| *B. adolescentis* | ATCC 15703^{T} | ATCC 15705 | |
| *B. angulatum* | DSM 20098^{T} | | |
| *B. animalis* | ATCC 25527^{T} | DSM 10140 | |
| *B. bifidum* | DSM 20456^{T} | NCIMB 8810 | |
| *B. boum* | ATCC 27917^{T} | | |
| *B. breve* | ATCC 15700^{T} | DSM 20091 | LMG 11613 |
| *B. catenulatum* | ATCC 27539^{T} | ATCC 27675 | |
| *B. dentium* | ATCC 27534^{T} | | |
| *B. gallicum* | DSM 20093^{T} | | |
| *B. gallinarum* | ATCC 33777^{T} | | |
| *B. infantis* | LMG 8811^{T} | | |
| *B. inopinatum* | DSM 10107^{T} | | |
| *B. longum* | ATCC 15707^{T} | | |
| *B. magnum* | ATCC 27540^{T} | | |
| *B. pseudocatenulatum* | DSM 20438^{T} | | |
| *B. pseudolongum* | ATCC 25526^{T} | | |
| *B. suis* | ATCC 27533^{T} | | |
| | | | |

| Other Strains | | | |
|---|---|---|---|
| *Bacillus cereus* | ATCC 11778 | | |
| *Bacteroides fragilus* | LMG 10263^{T} | | |
| *Brevibacterium casei* | ATCC 35513^{T} | | |
| *Clostridium difficile* | ATCC 9689^{T} | | |
| *Enterococcus feacalis* | DSM 20478^{T} | | |
| *Escherichia coli* | ATCC 35218 | | |
| *Lactobacillus acidophilus* | ATCC 4356^{T} | | |
| *Lactobacillus brevis* | LMG 18022 | | |
| *Lactobacillus bulgaricus* | ATCC 11842^{T} | | |
| *Lactobacillus casei* | ATCC 393^{T} | DSM 20011^{T} | |
| *Lactobacillus fermentum* | DSM 20052^{T} | | |
| *Lactobacillus plantarum* | DSM 20174^{T} | | |
| *Lactobacillus reuteri* | LMG 9213^{T} | | |
| *Lactobacillus rhamnosus* | ATCC 53103 | | |
| *Listeria monocytogenes* | ATCC 7644 | | |
| *Pediococcus acidilactici* | DSM 20284^{T} | | |
| *Propionibacterium avidum* | DSM 4901 | | |
| *Pseudomonas aeruginosa* | DSM 1117 | | |
| *Saccharomyces cerevisiae* | DSM 2548 | | |
| *Salmonella typhimurum* | ATCC 14028 | | |
| *Staphylococcus aureus* | ATCC 29213 | | |

| | | | |
|---|---|---|---|
| ATCC: American Type Culture Collection; DSM: Deutsche Sammlung von Mikroorganismen und Zellkulturen, Germany; LMG: Laboratory for Microbiology, University of Gent, Belgium; NCIMB: National Collections of Industrial and Marine Bacteria, UK. | | | |

All bifidobacteria strains were cultured in Mann Rogosa Sharp (MRS) broth (Oxoid, Basingstoke, UK) media at 37 °C for 24 hours under anaerobic conditions. The overnight cultures were stored at -20 °C until further processing.

DNA was extracted from bacterial cultures by thawing 5 ml of frozen overnight cultures in ice water. Subsequently, the cultures were centrifuged for 20 minutes at 4000 rpm at 4 °C (Sorvall RT7, Du Pont, Stevenage, UK) to pellet the bacterial cells. The pellets were washed with 1 ml TES (50 mM Tris-HCl [pH 8.0], 5 mM EDTA, 50 mM NaCl), followed by a centrifugation step of 10 minutes at 4000 rpm at 4 °C. Supernatants were removed and the pellet were resuspended in 1 ml of THMS (30 mM Tris-HCl [pH 8.0], 3 mM MgCl₂, 25% (w/v) sucrose). After transfer of the suspensions into a two ml eppendorf tube, 200 µl lysozyme (0.1 g/ml; Sigma Aldrich Chemie, Steinheim, DE) and 40 µl mutanolysine (1 mg/ml; Sigma Aldrich Chemie, DE) was added and incubated for 30 minutes at 37 °C. Subsequently, the solutions were centrifuged for 5 minutes at 10000 rpm at 4 °C (Sigma 1-15, Sigma Laborzentrifugen GmbH, Osterode am Harz, DE). Supernatants were removed and the pellets were resuspended in 100 µl THMS, whereto 400 µl TES (including 0.5% SDS) and 7.5 µl of Proteïnase K (20 mg/ml; Boehringer Mannheim GmbH, Mannheim, DE) were added. The mixture was vortexed and incubated for 30 minutes at 65 °C. Subsequently, a standard phenol/chloroform extraction was carried out, followed by a treatment with 2.5 µl RNase A (1 mg/ml; Roche Diagnostics, Mannheim, DE) for 30 minutes at 37 °C. Subsequently, the DNA was precipitated by storing at -20 °C for at least 30 minutes after addition of 2 volumes ice cold ethanol (96%) and 0.1 volume of 3 M sodium acetate (pH 5.2). Precipitated solutions were centrifuged for 20 minutes at 13000 rpm at 4 °C and the supernatants were washed with 500 µl 70% ethanol, followed by centrifugation at 13000rpm for 5 minutes at 4 °C. Supernatants were discarded and the pellets were air dried at room temperature. The DNA was resuspended in 100 µl sterile milli-Q and stored at -20 °C.

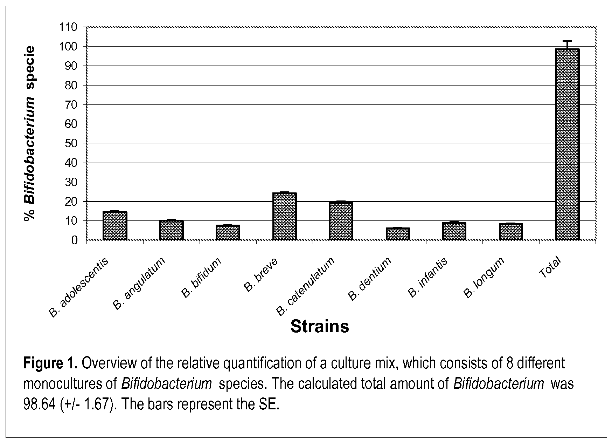

Firstly, the specificity of each duplex 5' nuclease assay was tested by performing a 25 µl amplification of the different strains (see table 2). These 25 µl PCR reactions were performed using 2.5 µl DNA template, 12.5 µl TaqMan Universal Master Mix (Applied Biosystems), 900 nM of each primer and 200 nM of each probe, followed by running the TaqMan Universal Temperature Profile, which consists of 2 minutes at 50 °C, 10 minutes at 95 °C, followed by 45 cycles of 15 seconds at 95 °C and 60 °C for 1 minute, on the ABI Prism 7700 (Applied Biosystems, Nieuwerkerk a/d IJssel, NL). All of the 5' nuclease assays were specific for the *Bifidobacterium* species for which they were developed and the 5'nuclease assay for determination of the total amount of *Bifidobacterium* detected all *Bifidobacterium* species tested, but no other strains like *Propionibacterium* or *Lactobacillus.* It should be noted that the 5'nuclease assay for *B. catenulatum* also detects *B. pseudocatenulatum.* Furthermore, DNAse and RNAse treated samples were tested to assure that no contaminated RNA was detected during the assay. Secondly, a mix of monocultures from *B. adolescentis, B. angulatum, B. breve, B. bifidum, B. catenulatum, B. dentium, B. infantis* and *B. longum* was prepared to verify that the total of this mix would sum up to approximately 100%. In that case, competition between the different *Bifidobacterium* species, which serve as template, can be excluded. This is indeed the case, as can be seen in figure 1, which shows the determined amounts of each Bifidobacterium species in the mix as well as the total amount of *Bifidobacterium* species in the mix.

The CV values for reproducibility and repeatability for the different kind of 5' nuclease assays were determined and can be found in table 3.

**Table 3**

| Sensitivity of the 5' nuclease assays in comparison to "conventional" PCR and reproducibility and repeatability of the 5' nuclease assays | | | |
|---|---|---|---|
| Target | Sensitivity^{a} (x) | Reproducibility^{b} [CV (%)] | Repeatability^{c} [CV (%)] |
| *B. adolescentis* | 10,000 | 5.11 | 5.68 |
| *B. angulatum* | 1000 | 19.48 | 20.92 |
| *B. bifidum* | 100 | 11.65 | 11.20 |
| *B. breve* | 100 | 2.06 | 4.08 |
| *B. catenulatum* | 1000 | 9.42 | 14.83 |
| *B. dentium* | 100 | 12.65 | 11.35 |
| *B. infantis* | 1000 | 2.34 | 2.31 |
| *B. longum* | 10,000 | 9.10 | 8.18 |

| | | | |
|---|---|---|---|
| ^{a} number of times that the 5' nuclease assay is more sensitive then "conventional" PCR ^{b} reproducibility is determined by testing monocultures (100%) in ten fold and calculation of the CV (%) based on the gained results ^{c} repeatability is determined by testing monocultures (100%) three times in four fold and calculation of the CV (%) based on results gained | | | |

The developed 5' nuclease assays were compared to the conventional qualitative species-specific PCR (using the primers as described by Matsuki, T., K. Watanabe, R. Tanaka, M. Fukuda, and H. Oyaizu. 1999. Distribution of bifidobacterial species in human intestinal microflora examined with 16S rRNA-gene-targeted species-specific primers. Appl. Environ. Microbiol. 65:4506-4512) to determine the sensitivity of the different assays as well as checking for false positive or negative results. Table 3 shows the different sensitivities of the 5' nuclease assays in relation to the conventional species specific PCR. Table 4 show the final optimal primer and probe concentrations used in the duplex 5' nuclease assays.

**Table 4**

| Optimised final primer and probe concentrations used in the different duplex 5' nuclease assays | | | | |
|---|---|---|---|---|
| **Target** | **5' nuclease assay** | **Forward Primer (nM)** | **Reverse Primer (nM)** | **Probe (nM)** |
| *B. adolescentis* | *B. adolescentis* | 300 | 150 | 100 |
| | All *Bifidobacterium* | 300 | 600 | 100 |
| *B. angulatum* | *B. angulatum* | 900 | 900 | 200 |
| | All *Bifidobacterium* | 300 | 300 | 50 |
| *B. bifidum* | *B. bifidum* | 600 | 600 | 200 |
| | All *Bifidobacterium* | 300 | 300 | 100 |
| *B. breve* | *B. breve* | 300 | 300 | 100 |
| | All *Bifidobacterium* | 450 | 450 | 150 |
| *B. catenulatum* | *B. catenulatum* | 300 | 300 | 100 |
| | All *Bifidobacterium* | 600 | 600 | 100 |
| *B. dentium* | *B. dentium* | 900 | 900 | 200 |
| | All *Bifidobacterium* | 300 | 300 | 50 |
| *B. infantis* | *B. infantis* | 300 | 300 | 100 |
| | All *Bifidobacterium* | 900 | 900 | 100 |
| *B. longum* | *B. longum* | 300 | 300 | 100 |
| | All *Bifidobacterium* | 600 | 600 | 200 |
| All | All *Bifidobacterium* | 450 | 450 | 100 |
| *Bifidobacterium* | All bacteria | 900 | 900 | 200 |

### Example 2: Clinical trial

The study was a double blind, placebo-controlled multi-center trial with two intervention groups. Fully formula fed infants, aged 28 to 90 days, were recruited from four hospitals in Germany. Infants were included in the study if they had a birth weight between 2600 and 4500 g, and were fully formula fed for at least four weeks before the start of the intervention period. Infants with congenital abnormalities, or with proven or suspected cow's milk allergy, infants derived from multiple births, infants that had received antibiotics less than two weeks before the start of the study, and infants that were fed any pro- or prebiotic formula less than a month before the start of the study, were excluded from the study. After enrolment, infants were randomly allocated to one of two treatment groups: a group receiving an infant formula supplemented with 0,8 g/100ml galacto-oligosaccharides and fructo-polysaccharides (GFSF-group) and a group receiving a standard infant formula (SF-group). The macronutrient composition of the formulas is shown in table 5.

**Table 5:**

| Macronutrient composition of the study formulas (per 100 ml ready to use formula) | | |
|---|---|---|
| | Carbohydrate mixture-supplemented formula (Aptamil 1 with GOS/FOS, Milupa) | Standard formula (Aptamil 1, Milupa) |
| Energy (kcal) | 72 | 72 |
| Protein (g) | 1.5 | 1.5 |
| Carbohydrate (g) | 8.5 | 8.5 |
| -Lactose (g) | 7.5 | 7.5 |
| -Starch (g) | 1 | 1 |
| Non-digestible oligosaccharides (g) | 0.8 | 0 |
| -Galacto-oligosaccharides (g) | 0.72 | 0 |
| -Fructo-polysaccharides (g) | 0.08 | 0 |
| Fat (g) | 3.6 | 3.6 |

A group of breast-fed infants was included as a reference group (BF group). Within three days after the start of the study period, after 4 weeks, and at the end of the study period (6 weeks), faecal samples were collected. The study was approved by the medical ethical committees of the four hospitals. Written informed consent was obtained from the parents before the start of the study.

Nucleic acids were isolated from faeces by thawing faecal samples in ice water, followed by a 10x (w/v) dilution in PBS (0.37 M NaCl, 2.7 mM KCl, 8.1 mM Na₂HPO₄ [pH 7.4]) and homogenisation for 10 minutes using a stomacher (IUL Instruments, Barcelona, Spain). Homogenised faeces was stored at -20°C prior to the actual DNA isolation. The extractions were started by thawing 1 ml of a homogenised faeces sample in ice water, followed by centrifugation for 1 minute at 1100 rpm to remove debris and large particles. Supernatants were transferred to a new tube and centrifuged for 5 minutes at 10000 rpm. Subsequently, the pellets were resuspended in 1 ml TN150 (10 mM Tris-HCl [pH 8.0], 10 mM EDTA) and transferred to sterile tubes containing 0.3 g zirconium beads (diameter 0.1 mm, BioSpec Products, Bartlesville, US). To these suspensions 150 µl of TE-buffered phenol (pH ± 7.5) was added and the samples were placed in a mini-bead beater (BioSpec Products, Bartlesville, US), for 3 minutes at 5000 rpm. After bead-beating the samples were immediately cooled on ice, before addition of 150 µl chloroform. Samples were vortexed shortly and centrifuged for 5 minutes at 10000 rpm, upper phases were transferred to clean 2 ml eppendorf tubes and the phenol/chloroform extraction was started. Phenol-chloroform extraction was followed by precipitation of DNA through placement of the samples at -20 °C for at least 30 minutes, after addition of 1 ml ice-cold ethanol (96%) and 50 µl 3 M sodium acetate (pH 5.2). Consecutively, the samples were centrifuged for 20 minutes at 13000 rpm and washed with 500 µl 70% ethanol. After centrifugation for 5 minutes at 13000 rpm, the supernatants were discarded and the pellets were air dried at room temperature. The DNA was resuspended in 100 µl sterile milli-Q and stored at -20 °C.

The duplex 5' nuclease assays are used for the relative quantification of the different *Bifidobacterium* species in faecal samples. The relative amount of each species is calculated according to Liu et. al. 2002. Briefly, efficiency of each amplification curve was calculated separately, by the formula E = (threshold _{A} / threshold _{B})^{-(Ct,A - Ct,B)} - 1. With help of the calculated efficiencies the initial amount of DNA (R₀) is calculated by Ro = threshold / (1 + E)^{Ct}. The initial amount of DNA of a *Bifidobacterium* species can then be divided with the initial amount of DNA of all *Bifidobacterium* species. Thereafter the obtained ratio's can be normalised with help of the ratio of a monoculture, which is set to 100%.

The total amount of *Bifidobacterium* was also determined with help of FISH, like earlier described (Langendijk, F. Schut, G. J. Jansen, G. C. Raangs, G. R. Kamphuis, M. H. Wilkinson and G. W. Welling "Quantitative fluorescence in situ hybridisation of Bifidobacterium spp. with genus-specific 16S rRNA-targeted probes and its application in fecal samples" Appl. Environ. Microbiol. 61(8):3069-75. (1995))

The percentage of the genus *Bifidobacterium* as a percentage of total bacteria was 75, 47, and 68% in the BF, SF, and GFSF group, respectively, which demonstrates that the GFSF group, fed a mixture of nondigestible carbohydrates, has a more bifidogenic flora, as in the BF group, than in the SF group.

In table 6 the prevalence of each species in the different groups at the beginning as well as at the end of the study is shown. In table 7 the percentage of *Bifidobacteria* species relative to the total amount of *Bifidobacteria* is shown.

**Table 6:**

| Prevalence (in %) of *Bifidobacteria* species in the faeces of infants after 6 weeks of feeding with human milk (BF), an infant formula with a prebiotic mixture (GFSF) or with a standard formula (SF). | | | |
|---|---|---|---|
| Species | BF | GFSF | SF |
| *B. catenulatum* | 80 | 67 | 75 |
| *B. adolescentis* | 20 | 11 | 50 |
| *B. breve* | 70 | 78 | 63 |
| *B. longum* | 50 | 56 | 63 |
| *B. bifidum* | 10 | 11 | 13 |
| *B. angulatum* | 30 | 11 | 13 |
| *B. infantis* | 100 | 100 | 100 |
| *B. dentium* | 20 | 11 | 13 |

**Table 7:**

| Percentage of *Bifidobacteria* species with respect to the total number of *Bifidobacteria* in the faeces after a 6 week feeding period. | | | |
|---|---|---|---|
| Species | Breast-fed % (sd) | GFSF-fed % (sd) | SF-fed % (sd) |
| *B. catenulatum* | 1.9 (1.0) | 1.5 (3.0) | 9.8 (12.6) |
| *B. adolescentis* | 0.3 (0.9) | 0.1 (0.2) | 2.9 (6.0) |
| *B. breve* | 11.7(9.6) | 5.4 (10.8) | 4.9 (10.7) |
| *B. longum* | 7.3 (13.9) | 5.4 (10.7) | 6.2(9.4) |
| *B. bifidum* | <0.1 (0.0) | <0.1 (0.0) | <0.1 (0.0) |
| *B. angulatum* | <0.0 (0.0) | <0.1 (0.2) | <0.1 (0.0) |
| *B. infantis* | 32.0 (18.9) | 32.1 (20.0) | 37.8(18.4) |
| *B. dentium* | <0.1 (0.0) | <0.1 (0.0) | <0.1 (0.0) |

A large variety of *Bifidobacterium* species is present in the three different groups. Furthermore, a significant decrease in prevalence and amount of *B. adolescentis* is visible in breast-fed infants and in infants receiving GFSF contrary to infants receiving a standard formula. After 6 weeks of feeding the prevalence and percentage of *B. adolescentis* is much higher in SF-fed babies than in babies which were GFSF or breast-fed. Analyses of the faecal samples of GFSF infants shows a large variety in the bifidobacterial flora similar to breast-fed infants and stimulation of only one or a few species is not observed. Besides the effect on *B. adolescentis* the profiles of breast-fed infants and infants receiving GFSF also showed less *B. catenulatum* (+ *B. pseudocatenulatum)* than the profile of infants receiving a standard formula. *B. infantis,* and *B. longum* seems to be predominant in breast-fed infants as well as in infants receiving a standard formula (SF) or a standard formula supplemented with prebiotics (GFSF). Also *B. breve* was dominant in all three groups, but in the group receiving breast milk *B. breve* as a % of total bifidobacteria was higher (11.7 %) as in the SF (4.9 %) and GFSF (5.4%) group.

### Example 3: Animal experiments on allergy

Specific pathogen free male BALB/c mice were obtained from Charles River (Maastricht, the Netherlands). Food and water was provided *ad libitum* and the mice were used when 6-9 weeks of age. All experiments were approved by the animal ethics committee of the University of Utrecht, The Netherlands.

Ovalbumin (grade V) and acetyl-β-methylcholine chloride (methacholine) were purchased from Sigma Chemical Co. (St. Louis, MO, USA). Aluminium hydroxide (AlumImject) was purchased from Pierce (Rockford, IL, USA).

Mice were sensitised by two i.p. injections with 10 µg ovalbumin adsorbed onto 2.25 mg aluminium hydroxide in 100 µl saline or saline alone on days 0 and 7. Mice were challenged on days 35, 38, and 41 by inhalation of ovalbumin aerosols in a plexiglass exposure chamber for 20 minutes. The aerosols were generated by nebulising an ovalbumin solution (10 mg/ml) in saline using a Pari LC Star nebulizer (Pari respiratory Equipment, Richmond, VA, USA).

Mice were treated daily with 1x10e9 (CFU) *Bifidobacterium breve* and 25 mg of a mixture of galactooligosaccharides and fructopolysaccharides (9:1) orally via gavage (0.2 ml, physiological salt solution) starting at day 28 upto the end of the experiment (i.e. day 42). As a control 0.2 ml physiological salt solution was administered via gavage.

Airway responsiveness to inhaled nebulised methacholine was determined 24 hours after the final aerosol challenge, in conscious, unrestrained mice using whole body plethysmo-graphy (BUXCO, EMKA, Paris, France). The airway response was expressed as enhanced pause (PenH).

Statistical Analysis: The airway response curves to methacholine were statistically analysed by a general linear model or repeated measurements followed by post-hoc comparison between groups. Cell counts were statistically analysed using the Mann-Whitney U test (Siegel, S., Castellan Jr. N J, 1988, "Nonparametric statistics for the behavioural sciences" 2nd ed. McGraw Hill Book Company, New York, USA). All other analyses were performed using Student's t-test (Abramowitz, M., Stegun, I.A., 1972, "Handbook of mathematical functions" Dover publications, Inc. New York, USA). A probability value of p<0.05 was considered as statistically significant.

Results on airway hyperresponsiveness: Measurements on airway hyperresponsiveness show that compared to control the mice receiving the *B. breve* + a mixture of galactooligosaccharides and fructopolysaccharides show a statistically reduced airway hyperresponsiveness, indicative of a lowered asthmatic reaction.

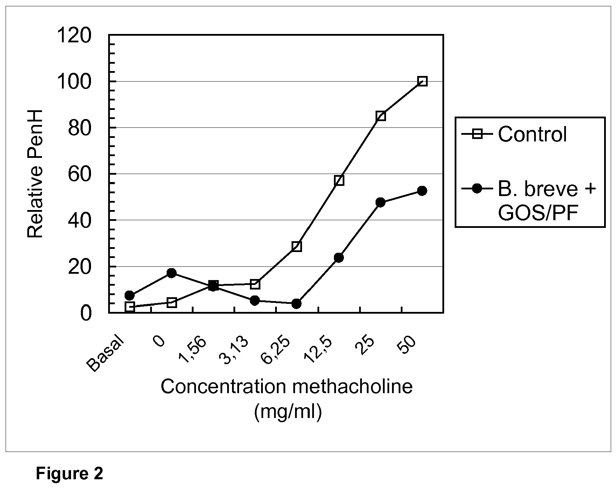

In figure 2 the airway hyperresponsiveness is plotted as relative PenH (enhanced pause) versus the metacholine concentration for mice receiving a combination of *B. Breve* + a mixture of GOS/FOS and a control group of mice receiving saline instead. The plotted values of relative PenH are obtained after subtraction of the blank values obtained for mice not ovalbumin-sensitised and normalisation to the value obtained for the control group at the highest concentration of metacholine.

The compositions of all following examples may additionally contain minerals, trace elements and vitamins, choline, taurine, carnitine, and/or myo-inositol or mixtures thereof, as known in the art and in accordance with international guidelines. Furthermore, organic acids, flavours and or colorants may or may not be present.

### Example 4

An infant milk formula containing per 100 ml final product (and per 13.1 g powder):

| | |
|---|---|
| 8 energy % protein | 1.4 g (casein whey mixture) |
| 45 energy % digestible carbohydrates | 7.5 g |
| 47 energy % fat | 3.5 g |
| GOS (90% galacto-oligosaccharides, Borculo Domo NL) / polyfructose, (10% inulin, Raftilin HP, Orafti BE) | 0.4 g |
| *B. breve:* | 1.3x10⁸ cfu |

### Example 5

An infant milk formula containing per 100 ml final product (and per 14 g powder):

| | |
|---|---|
| 10 energy % protein | 1.8 g (casein whey mixture) |
| 46 energy % digestible carbohydrates | 8.0 g |
| 44 energy % fat | 3.4 g |
| GOS/polyfructose (see ex. 4) | 0.4 g |
| *B. breve* | 1.4x10⁸ cfu |

### Example 6

An infant milk formula containing per 100 ml final product (and per 15 g powder):

| | |
|---|---|
| 10 energy % protein | 1.7 g (casein whey mixture) |
| 48 energy % digestible carbohydrates | 8.1 g |
| 42 energy % fat | 3.1 g |
| GOS/polyfructose 9/1 | 0.8 g |
| Galactomannan | 0.42 |
| *B. breve* | 1.5x10⁸ cfu |

### Example 7

An infant milk formula containing per 100 ml final product (and per 15.9 g powder):

| | |
|---|---|
| 13 energy % protein | 2.2 g (casein whey mixture) |
| 49 energy % digestible carbohydrates | 8.6 g |
| 37 energy % fat | 3.0 g |
| GOS/polyfructose 9/1 | 0.8 g |
| Galactomannan | 0.4 g |
| *B. breve* | 1.6x10⁸ cfu |

### Example 8

An infant milk formula containing per 100 ml final product (and per 13.5 g powder):

| | |
|---|---|
| 9 energy % protein equivalent | 1.5 g (hydrolysed whey protein) |
| 42 energy % digestible carbohydrates | 6.9 g |
| 49 energy % fat | 3.6 g |
| GOS/polyfructose/sialyllactose 7/2/1 | 0.8 g |
| *B. breve* | 1.4x10⁸ cfu |

### Example 9

An infant formula containing per 100 ml final product (and per 13.5 g powder):

| | |
|---|---|
| 11 energy % protein | 1.8 g (soy protein) |
| 40 energy % digestible carbohydrates | 6.7 g |
| 49 energy % fat | 3.6 g |
| GOS/galacto-ogosaccharides/polyfructose 8/1/1 | 0.8 g |
| *B. breve* | 1.4x10⁸ cfu |

### Example 10

A supplement containing: 0.4-0.8 g to be added to 100 ml milk: per g:

| | |
|---|---|
| 0.26 g | galactomannan, |
| 0.44 g | digestible carbohydrates |
| 0.3 g | GOS/polyfructose 85/15 |
| 1.0x10⁹ cfu | *B. breve* |

### Example 11

An infant nutrition containing per 100 g (85 g to be added to 240 ml milk)

| | |
|---|---|
| 4 energy % protein | 4.7 g (cow's milk protein) |
| 53 energy % digestible carbohydrates | 68 g |
| 43 energy % fat | 24.6 g |
| GOS/polyfructose 9/1 | 0.8 g |
| *B. breve* | 1.2x10⁹ cfu |

### Example 12

An infant nutrition containing per 100 ml product

| | |
|---|---|
| 11 energy % protein | 2.8 g (casein) |
| 49 energy % carbohydrates | 12.3 g |
| 40 energy % fat | 4.4 g |
| GOS/polyfructose 85/15 | 0.8 g |
| *B. breve* | 5x10⁸ cfu |

### Example 13

An infant nutrition composed of rice flour containing per 100 g dry product: (4-7 spoons to be added to 200 ml warm infant formula, follow-on formula, toddler's milk or cow's milk)

| | |
|---|---|
| 7.4 g | protein (vegetable) |
| 83 g | carbohydrates |
| 0.5 g | fat |
| 3 g | dietary fibre including 1.5 g GOS/polyfructose 9/1 |
| 1x10¹⁰ | cfu *B. breve* |

### Example 14

An infant nutrition composed of precooked flakes (wheat, rye, rice, barley, maize, oat, buckwheat) containing per 100 g dry product. (5-7 spoons to be added to 250 ml warm infant formula, follow-on formula, toddler's milk or cow's milk)

| | |
|---|---|
| 9.5 g | protein (vegetable) |
| 74 g | carbohydrates |
| 2.0 g | fat |
| 3 g | dietary fibre including 1.5 g GOS/polyfructose 8/2 |
| 2x10¹⁰ | cfu *B. breve* |

## Claims

1. Use of a preparation comprising *Bifidobacterium breve* and a mixture of at least two non-digestible soluble carbohydrate components A and B, wherein carbohydrate component A constitutes 95 to 80 wt% of the sum of carbohydrate component A and carbohydrate component B and carbohydrate component B constitutes 5 to 20 wt% of the sum of carbohydrate component A and carbohydrate component B, with A + B = 100 wt% components A and B differing
(i) in the number of monosaccharide units of the carbohydrate, component A having an average chain length which is at least 5 monosaccharide units lower than the average chain length of component B, and
(ii) in the monosaccharide units of the carbohydrate,
for the manufacture of a composition for the prevention or treatment of immune mediated diapers rashes,
wherein 80 to 100 wt% of carbohydrate components A belong to the group of galacto-oligosaccharides and 80 to 100 wt% of carbohydrate components B belong to the group of fructo-polysaccharides, and in which at least 50% of the total non-digestible soluble carbohydrates is selected from disaccharides to eicosasaccharides.

2. The use according to claim 1, in which more than 95% total soluble non-digestible carbohydrates has a chain length of no more than 100 units.

3. The use according to claim 1 or 2, wherein the average chain length of carbohydrate component A is between 2 and 6 units, and the average chain length of carbohydrate component B is between 7 and 30 units.

4. The use according to any one of claims 1-3, wherein the carbohydrate mixture is composed of galacto-oligosaccharide and inulin.

5. The use according to any one of claims 1-4, wherein the composition comprises 10⁷ to 10¹¹ cfu of *Bifidobacterium breve* per g of total non-digestible soluble carbohydrate.

6. The use according to any one of claims 1-5 wherein the composition is for use as a supplement, wherein the probiotic *Bifidobacterium breve* is present in the supplement in an amount of from 1x10⁶ to 1.5x10¹¹ cfu/g, calculated on the basis of the supplement.

7. The use according to any one of claims 1-5, wherein the composition is for use as an infant nutrition, said composition further comprising digestible carbohydrate, a lipid source, and a protein source.

8. The use according to claim 7, wherein the protein is in hydrolysate form.

9. The use according to claim 8, wherein the composition is for the treatment or prevention of immune mediated diapers rashes in infants at risk of allergy.

## Patentansprüche

1. Verwendung eines Präparats, das *Bifidobacterium breve* und ein Gemisch von wenigstens zwei unverdaulichen löslichen Kohlenhydratkomponenten A und B umfasst,
wobei Kohlenhydratkomponente A 95 bis 80 Gew.% der Summe von Kohlenhydratkomponente A und Kohlenhydratkomponente B ausmacht und Kohlenhydratkomponente B 5 bis 20 Gew.% der Summe von Kohlenhydratkomponente A und Kohlenhydratkomponente B ausmacht, wobei A + B = 100 Gew.%, Komponenten A und B, die sich voneinander unterscheiden
(i) in der Anzahl der Monosaccharideinheiten des Kohlenhydrats, wobei Komponente A eine mittlere Kettenlänge aufweist, die um wenigstens 5 Monosaccharideinheiten geringer ist als die mittlere Kettenlänge von Komponente B, und
(ii) in den Monosaccharideinheiten des Kohlenhydrats,
zur Herstellung einer Zusammensetzung zur Prävention oder Behandlung von immunvermittelten Windelausschlägen;
wobei 80 bis 100 Gew.-% der Kohlenhydratkomponenten A zu der Gruppe der Galacto-Oligosaccharide gehören und 80 bis 100 Gew.-% der Kohlenhydratkomponenten B zu der Gruppe der Fructo-Oligosaccharide gehören und wobei wenigstens 50% der gesamten unverdaulichen löslichen Kohlenhydrate aus Disacchariden bis Eicosasacchariden ausgewählt sind.

2. Verwendung gemäß Anspruch 1, wobei mehr als 95% der gesamten löslichen unverdaulichen Kohlenhydrate eine Kettenlänge von nicht mehr als 100 Einheiten aufweisen.

3. Verwendung gemäß Anspruch 1 oder 2, wobei die mittlere Kettenlänge der Kohlenhydratkomponente A 2 bis 6 Einheiten beträgt und die mittlere Kettenlänge der Kohlenhydratkomponente B 7 bis 30 Einheiten beträgt.

4. Verwendung gemäß einem der Ansprüche 1 bis 3, wobei das Kohlenhydratgemisch aus Galacto-Oligosaccharid und Inulin zusammengesetzt ist.

5. Verwendung gemäß einem der Ansprüche 1 bis 4, wobei die Zusammensetzung 10⁷ bis 10¹¹ kbE *Bifidobacterium breve* pro g der gesamten unverdaulichen löslichen Kohlenhydrate umfasst.

6. Verwendung gemäß einem der Ansprüche 1 bis 5, wobei die Zusammensetzung zur Verwendung als Ergänzung dient, wobei das probiotische *Bifidobacterium breve* in der Ergänzung in einer Menge von 1 x 10⁶ bis 1,5 x 10¹¹ kbE/g vorhanden ist, berechnet auf der Basis der Ergänzung.

7. Verwendung gemäß einem der Ansprüche 1 bis 5, wobei die Zusammensetzung zur Verwendung als Säuglingsnahrung dient, wobei die Zusammensetzung weiterhin verdauliches Kohlenhydrat, eine Lipidquelle und eine Proteinquelle umfasst.

8. Verwendung gemäß Anspruch 7, wobei das Protein in Hydrolysatform vorliegt.

9. Verwendung gemäß Anspruch 8, wobei die Zusammensetzung zur Behandlung oder Prävention von immunvermittelten Windelausschlägen bei Säuglingen dient, bei denen die Gefahr einer Allergie besteht.

## Revendications

1. Utilisation d'une préparation comprenant *Bifidobacterium breve* et un mélange d'au moins deux composants glucidiques solubles non digestibles A et B,
dans laquelle le composant glucidique A représente 95 à 80 % en poids de la somme du composant glucidique A et du composant glucidique B et le composant glucidique B représente 5 à 20 % en poids de la somme du composant glucidique A et du composant glucidique B, avec A + B = 100 % en poids % de composants A et B qui diffèrent
(i) concernant le nombre d'unités de monosaccharide du glucide, le composant A ayant une longueur de chaîne moyenne qui est au moins inférieure de 5 unités de monosaccharide par rapport à la longueur de chaîne moyenne du composant B, et
(ii) concernant les unités de monosaccharide du glucide,
pour la fabrication d'une composition pour la prévention ou le traitement d'éruptions cutanées dues aux couches à médiation immunitaire,
dans laquelle 80 à 100 % en poids de composants glucidiques A appartiennent au groupe des galacto-oligosaccharides et 80 à 100 % en poids de composants glucidiques B appartiennent au groupe des fructo-polysaccharides, et dans laquelle au moins 50 % du total des glucides solubles non digestibles sont choisis des disaccharides aux eicosasaccharides.

2. Utilisation selon la revendication 1, dans laquelle plus de 95 % du total des glucides solubles non digestibles ont une longueur de chaîne d'au plus 100 unités.

3. Utilisation selon la revendication 1 ou 2, dans laquelle la longueur de chaîne moyenne du composant glucidique A est comprise entre 2 et 6 unités, et la longueur de chaîne moyenne du composant glucidique B est comprise entre 7 et 30 unités.

4. Utilisation selon l'une quelconque des revendications 1 à 3, dans laquelle le mélange de glucides est composé de galacto-oligosaccharide et d'inuline.

5. Utilisation selon l'une quelconque des revendications 1 à 4, dans laquelle la composition comprend 10⁷ à 10¹¹ cfu de *Bifidobacterium breve* par g de glucide soluble non digestible total.

6. Utilisation selon l'une quelconque des revendications 1 à 5, dans laquelle la composition est destinée à être utilisée comme supplément, dans laquelle le probiotique *Bifidobacterium breve* est présent dans le supplément en une quantité de 1x10⁶ à 1,5x10¹¹ cfu/g, calculée sur la base du supplément.

7. Utilisation selon l'une quelconque des revendications 1 à 5, dans laquelle la composition est destinée à être utilisée en tant qu'alimentation infantile, ladite composition comprenant en outre un glucide digestible, une source de lipide et une source de protéine.

8. Utilisation selon la revendication 7, dans laquelle la protéine est sous forme d'hydrolysat.

9. Utilisation selon la revendication 8, dans laquelle la composition est destinée au traitement ou à la prévention d'éruptions cutanées dues aux couches à médiation immunitaire chez des nourrissons présentant un risque d'allergie.


### SEQUENCE LISTING

<110> N.V. Nutricia
<120> Synbiotic composition for infants
<130> P208356EP1
<140> EP08167964.9
   <141> 2004-10-25
<160> 27
<170> PatentIn version 3.1
<210> 1
   <211> 18
   <212> DNA
   <213> Artificial Sequence
<220>
   <223> primer
<400> 1
   atagtggacg cgagcaag 18
<210> 2
   <211> 12
   <212> DNA
   <213> Artificial Sequence
<220>
   <223> primer
<400> 2
   agattgaaga gt 12
<210> 3
   <211> 32
   <212> DNA
   <213> Artificial Sequence
<220>
   <223> primer
<400> 3
   ttggcgaaat cgctgaaaga acgtttcttt tt 32
<210> 4
   <211> 18
   <212> DNA
   <213> Artificial Sequence
<220>
   <223> primer
<400> 4
   tggtggtttg agaactgg 18
<210> 5
   <211> 15
   <212> DNA
   <213> Artificial Sequence
<220>
   <223> primer
<400> 5
   atagtgtcga cgaac 15
<210> 6
   <211> 30
   <212> DNA
   <213> Artificial Sequence
<220>
   <223> primer
<400> 6
   aacaataaac aaaacaaagg ccaaagcctc 30
<210> 7
   <211> 18
   <212> DNA
   <213> Artificial Sequence
<220>
   <223> primer
<400> 7
   gttgatttcg ccggactc 18
<210> 8
   <211> 12
   <212> DNA
   <213> Artificial Sequence
<220>
   <223> primer
<400> 8
   ttcgcaagcc ta 12
<210> 9
   <211> 25
   <212> DNA
   <213> Artificial Sequence
<220>
   <223> primer
<400> 9
   tcgcgcaaaa actccgctgg caaca 25
<210> 10
   <211> 18
   <212> DNA
   <213> Artificial Sequence
<220>
   <223> primer
<400> 10
   gtggtggctt gagaactg 18
<210> 11
   <211> 14
   <212> DNA
   <213> Artificial Sequence
<220>
   <223> primer
<400> 11
   gatagcaaaa cgat 14
<210> 12
   <211> 36
   <212> DNA
   <213> Artificial Sequence
<220>
   <223> primer
<400> 12
   cgaaacaaac actaaatgat tcctcgttct tgctct 36
<210> 13
   <211> 17
   <212> DNA
   <213> Artificial Sequence
<220>
   <223> primer
<400> 13
   gtggacgcga gcaatgc 17
<210> 14
   <211> 18
   <212> DNA
   <213> Artificial Sequence
<220>
   <223> primer
<400> 14
   aatagagcct ggcgaaat 18
<210> 15
   <211> 20
   <212> DNA
   <213> Artificial Sequence
<220>
   <223> primer
<400> 15
   cgaagcaaac gatgacatca 20
<210> 16
   <211> 16
   <212> DNA
   <213> Artificial Sequence
<220>
   <223> primer
<400> 16
   ccgccaccca cagtct 16
<210> 17
   <211> 18
   <212> DNA
   <213> Artificial Sequence
<220>
   <223> primer
<400> 17
   agcaaaggga aacaccat 18
<210> 18
   <211> 18
   <212> DNA
   <213> Artificial Sequence
<220>
   <223> primer
<400> 18
   gtttacgcgt ccaacgga 18
<210> 19
   <211> 18
   <212> DNA
   <213> Artificial Sequence
<220>
   <223> primer
<400> 19
   cgcgagcaaa acaatggt 18
<210> 20
   <211> 10
   <212> DNA
   <213> Artificial Sequence
<220>
   <223> primer
<400> 20
   taacgatcga 10
<210> 21
   <211> 35
   <212> DNA
   <213> Artificial Sequence
<220>
   <223> primer
<400> 21
   aacgaacaat agagttttcg aaatcaacag caaaa 35
<210> 22
   <211> 18
   <212> DNA
   <213> Artificial Sequence
<220>
   <223> primer
<400> 22
   tggaagacgt cgttggct 18
<210> 23
   <211> 11
   <212> DNA
   <213> Artificial Sequence
<220>
   <223> primer
<400> 23
   ttatcgcgcc a 11
<210> 24
   <211> 20
   <212> DNA
   <213> Artificial Sequence
<220>
   <223> primer
<400> 24
   ggcaaaacgc acccaccgca 20
<210> 25
   <211> 18
   <212> DNA
   <213> Artificial Sequence
<220>
   <223> primer
<400> 25
   gggatgctgg tgtggaag 18
<210> 26
   <211> 12
   <212> DNA
   <213> Artificial Sequence
<220>
   <223> primer
<400> 26
   agatgctcgc gt 12
<210> 27
   <211> 30
   <212> DNA
   <213> Artificial Sequence
<220>
   <223> primer
<400> 27
   ccactatcca gttcaaacca ccacgcgcca 30